Europäisches Patentamt

European Patent Office

Office européen des brevets

(11) Publication number : **0 509 831 A1**

# EUROPEAN PATENT APPLICATION

(21) Application number : 92303465.6

(22) Date of filing : 16.04.92

(51) Int. Cl.$^5$: **C12N 15/16**, A61K 37/43, C07K 15/00

(30) Priority : **19.04.91 US 688354**

(43) Date of publication of application :
**21.10.92 Bulletin 92/43**

(84) Designated Contracting States :
**AT BE CH DE DK ES FR GB GR IT LI LU NL PT SE**

(71) Applicant : **ELI LILLY AND COMPANY**
**Lilly Corporate Center**
**Indianapolis Indiana 46285 (US)**

(72) Inventor : **Bierman, Kristin Lynne**
**887 Leatherwood Drive**
**Greenwood, Indiana 46143 (US)**
Inventor : **Chan, Ging**
**318 West 24th Place**
**Chicago, Illinois 60616 (US)**
Inventor : **Heiman, Mark Louis**
**5740 Susan Drive East**
**Indianapolis, Indiana 46250 (US)**
Inventor : **Hsiung, Hansen Maxwell**
**4760 Cole Porter Lane**
**Indianapolis, Indiana 46032 (US)**

(74) Representative : **Hudson, Christopher Mark et al**
**Erl Wood Manor**
**Windlesham Surrey GU20 6PH (GB)**

(54) Precursor form of bovine growth hormone releasing factor and related DNA compounds.

(57)    The present invention provides polypeptide compounds which are two forms of the bovine growth hormone releasing factor (bGRF) precursor and the bGRF leader peptide, and a new form of mature bGRF. The invention also provides DNA compounds encoding these polypeptide compounds and mature bGRF, pharmaceutical compositions and methods for the inducement of growth hormone release.

EP 0 509 831 A1

Jouve, 18, rue Saint-Denis, 75001 PARIS

Growth hormone releasing factor (GRF) is useful to stimulate the production of growth hormone in humans and animals. Bovine GRF (bGRF) is of particular interest for veterinary use. The amino acid sequence of mature bGRF has been elucidated. Esch et al., BBRC 117:772-779 (1983). However, the DNA sequence encoding bGRF in nature was unknown until the present invention. Provided herein are previously unknown polypeptide compounds which comprise two forms of the bGRF precursor and the bGRF peptide. Also included are DNA compounds encoding polypeptide compounds of the invention and mature bGRF.

The present invention provides DNA compounds that comprise DNA sequences encoding bovine growth hormone releasing factor, bGRF precursor, and the bGRF leader peptide. Also provided are polypeptide compounds including the bGRF precursor in addition to the bGRF leader peptide linked to the bGRF precursor.

The polypeptide compounds of the invention are SEQ ID No. 4 or 6 and the pharmaceutically acceptable acid or carboxylic acid addition salts thereof.

The DNA compounds of the invention comprise DNA sequences encoding mature bGRF, the bGRF precursor, and the bGRF leader peptide. A preferred DNA sequence encoding mature bGRF is SEQ ID No. 1.

A preferred DNA sequence encoding the bGRF precursor is SEQ ID No. 3.

A preferred DNA sequence encoding the bGRF precursor preceded by its leader peptide sequence is SEQ ID No. 5.

Also provided by the present invention are pharmaceutical compositions where the active ingredient is a polypeptide compound comprising the bGRF precursor. Finally, the invention provides methods for inducing growth hormone release, which comprises administering polypeptide compounds comprising the bGRF precursor. The compositions and methods may be useful for inducing growth hormone release in a variety of animals, including humans, pigs, cattle, sheep, chickens and fish. The preferred uses of the compositions and methods are in cattle.

The following section provides a more detailed description of the present invention. For purposes of clarity and as an aid in the understanding of the invention, as disclosed and claimed herein, the following terms and abbreviations are defined below.

Definitions

GRF - a polypeptide with growth hormone releasing factor activity.

$GRF(1-29)NH_2$ - the minimal portion of GRF that is required for full potency.

High-Level Expression in E coli - the production of a GRF analog encoded by a cloned gene at levels such that the gene product is detectable by Coomassie Blue staining.

Isolated DNA Sequence - any DNA sequence, however constructed or synthesized, which is locationally distinct from its natural location in genomic DNA. The definition includes the isolated DNA sequence in all its forms other than the natural state. For example, the DNA sequence may be inserted into a plasmid or phage vector or inserted into the genome of the organism from which it came or any other organism to increase the gene dosage.

Mature bGRF - bGRF (1-44); SEQ ID No. 2

bGRF precursor - bGRF (1-76); SEQ ID No. 4

Brief Description of the Drawings

The figure provided herein is not drawn exactly to scale.

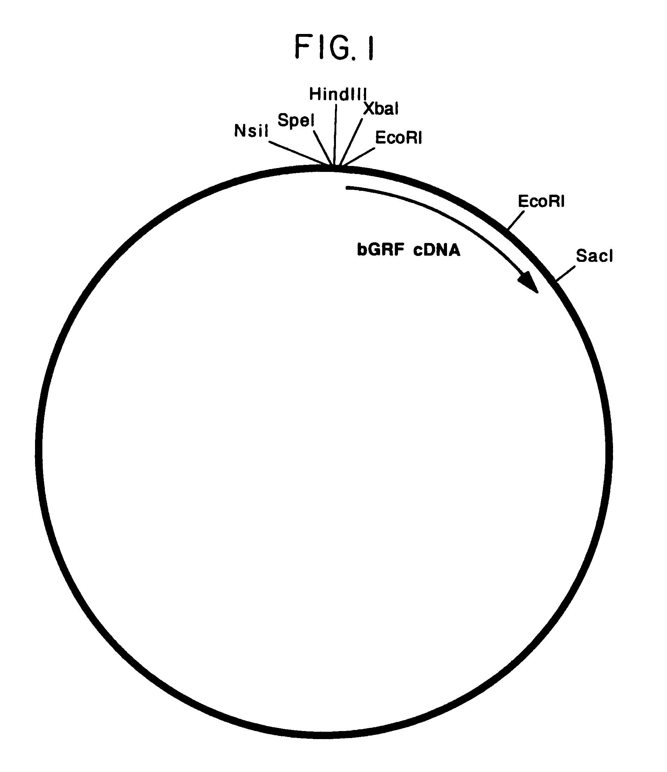

Figure 1 - A restriction site and function map of plasmid pHS507.

Detailed Description of the Invention

The present invention provides isolated DNA compounds which comprise DNA sequences encoding mature growth hormone releasing factor, the bGRF precursor, and the bGRF leader peptide linked to the bGRF precursor. The invention also provides polypeptide compounds.

Although the amino acid structure of a form of mature bGRF has been previously elucidated Esch et al., Biochem. Biophys. Res. Commun. 177:772 (1983), the present invention provides the heretofore unknown bGRF precursor. The precursor compound is represented by SEQ ID No. 4 and the pharmaceutically acceptable acid or carboxylic acid addition salts thereof. The bGRF precursor is especially useful because it fills a need in the art for a compound with bGRF activity that can be produced at high levels in E. coli. The compounds are of sufficient size that the serious degradation problem encountered with expression of mature GRF in E. coli is escaped. See, e.g., Kirschner et al., J. Biotech. 12:247, 248 (1989).

Another polypeptide compound of the invention is bGRF precursor with the bGRF leader peptide attached

EP 0 509 831 A1

to the precursor's amino terminus. The compound is represented by the SEQ ID No. 6 and the pharmaceutical acceptable acid or carboxylic acid addition salts thereof.

The SEQ ID. No. 6 compound is particularly useful when produced in mammalian cells. The leader peptide causes secretion of the polypeptide product; cleavage of the leader peptide occurs as the product is secreted.

All of the peptide compounds of the invention can be made by chemical methods well-known in the art, including solid phase peptide synthesis or recombinant methods. Both methods are described in U.S. Patent 4,617,149, herein incorporated by reference. Recombinant methods are preferred if a high yield is desired. A general method for the construction of any desired DNA sequence is provided in Brown et al., Methods in Enzymology 68:109 (1979), herein incorporated by reference.

The DNA sequences can be synthesized using automated DNA synthesizers, such as the ABS (Applied Biosystems, 850 Lincoln Centre Drive, Foster City, CA 94404) 380B DNA synthesizer. The DNA sequences can also be generated by the polymerase chain reaction. See, e.g., European Patent Publication No. 0258017, published March 2, 1988.

The amino acid sequences of the polypeptide compounds of the invention can be encoded by a multitude of different DNA sequences because most of the amino acids are encoded by more than one DNA triplet. Because these alternate DNA sequences would encode the same amino acid sequences, the present invention further comprises these alternate sequences.

Skilled artisans recognize that the initiation of translation of polypeptides in E. coli is via a methionine residue. The polypeptides of the invention do not begin with methionine. Therefore, the compounds may be produced in several ways. After expression of the polypeptide compound, a leader sequence of methionine or methionine and additional amino acids are removed by any of several well-known routes. See, e.g., U.S. Patents 4,745,069 and 4,782,139; Villa et al., Eur. J. Biochem. 171:137 (1988); Geli et al., Gene 80:129 (1989); and European Patent Publication No. 0199018.

Other routes of production are well-known to skilled artisans. For example, the compounds are produced in eucaryotic cells using SV40-derived vectors. Expression in eucaryotic cells is achieved via the bGRF precursor cDNA sequence. Such a method is described in U.S. Patent 4,775,624. Several alternate methods of expression are described in J. Sambrook, E.F. Fritsch & T. Maniatis, Molecular Cloning: A Laboratory Manual 16.3-17.44 (1989). Gene expression in Saccharomyces cerevisiae is detailed in 1 Current Protocols in Molecular Biology (F.M. Ausubel, R. Brent, R.E. Kingston, D.D. Moore, J.G. Seidman, J.A. Smith & K. Struhl 1989).

The DNA sequences of the present invention encode the polypeptide-compounds of the present invention and mature bGRF. The amino acid sequence of mature bGRF is known but the DNA sequence encoding it was unknown until the present invention.

Included in the polypeptide compounds of this invention are their pharmaceutically acceptable non-toxic acid addition salts and their pharmaceutically acceptable non-toxic carboxylic acid salts.

The term "pharmaceutically acceptable non-toxic acid addition salts" encompasses both organic and inorganic acid addition salts including, for example, those prepared from acids such as hydrochloric, hydrofluoric, sulfuric, sulfonic, tartariac, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. Preferably, the acid addition salts are those prepared from hydrochloric acid, acetic acid, or succinic acid. Any of the above salts is prepared by conventional methods.

The term "carboxylic acid salts" includes, for example, amine, ammonium, quaternary ammonium, alkali metal and alkaline earth metal salts such as calcium, magnesium, sodium, potassium, and lithium, and the like.

The present invention also provides a pharmaceutical composition comprising as the active agent a polypeptide compound or a pharmaceutically acceptable acid or carboxylic acid addition salt thereof, wherein said polypeptide compound is SEQ ID No. 4 and a pharmaceutically acceptable solid or liquid carrier.

Also provided in the present invention is a method for inducing growth hormone release which comprises administering an effective amount of a polypeptide compound represented by SEQ ID No. 4 and the pharmaceutically acceptable acid or carboxylic acid addition salts thereof.

Doses of the precursor compounds of this invention are administered to the recipient for a period during which stimulation of the release of growth hormone is desired. The weight of the recipient and mode of administration will have an influence upon the size of the dose necessary to induce a particular response. Preferred doses in cattle are estimated to be about 3 mg/day.

In administering the polypeptide compounds of this invention parenterally, the pharmaceutical forms suitable for injection include sterile aqueous solutions or dispersions and sterile powders for reconstitution into sterile injectable solutions or dispersions. The carrier can be a solvent or dispersing medium containing, for example, water, ethanol, polyol (for example glycerol, propylene glycol, liquid polyethylene glycol, and the like), suitable mixtures thereof, or vegetable oils. Proper fluidity can be maintained, for example, by the use of a coating such as lecithin, by the maintenance of the required particle size in the case of dispersion and by the use of surfactants. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin.

Sterile injectable solutions can be prepared by incorporating the compounds of this invention in the required amount of the appropriate solvent with various of the other agents, as desired. If desired, and for more effective distribution, the compounds can be incorporated into slow release or targeted delivery systems such as polymer matrices, liposomes, and microspheres. The compounds may be delivered via mechanical release devices, osmotic pumps, or any other release device or system which provides continuous or pulsatile delivery. Because the art of the pharmaceutical administration of peptide compounds is in its infancy, it is anticipated that significant advances in this technology will occur. The compounds of the invention will be useful as administered by these new methods.

It is especially advantageous to formulate the compounds of this invention in unit dosage form for ease of administration and uniformity of dosage. Unit dosage form as used herein refers to physically discrete units suited as unitary dosages for the subject to be treated. Each unit contains a predetermined quantity of the compound calculated to produce the desired therapeutic effect in association with the pharmaceutically acceptable carrier. The specific unit dosage form is dictated by and directly dependent upon (a) the unique characteristics of the particular composition and (b) the particular therapeutic effect to be achieved.

The following examples are illustrative of this invention. They are not intended to be limiting upon the scope thereof.

Example 1

Expression in E. coli of a bGRF Precursor

Plasmid pHS507 is available from the NRRL in E. coli strain DH5α/pHS507 under accession number B-18766 (date of deposit: February 9, 1991). The bGRF precursor is expressed using the procedures of U.S. Patent 4,828,988, herein incorporated by reference.

Example 2

Purification of the bGRF Precursor

A cation exchange column is prepared using S Sepharose® (Pharmacia, 800 Centennial Ave., Piscataway, NJ 08854) resin. The column contains one liter of resin per 50 g of material. The bGRF precursor containing material is added to the column at a flow rate of 0.1 1/cm$^2$/hr and washed with 2 column volumes of 0.1 M sodium chloride in 0.05 N acetic acid-7M urea. The polypeptide is eluted by a linear gradient of 0.25 M to 1.6 M sodium chloride in acetic acid-urea using three column volumes of each with 0.1 column volume fractions collected. The polyppeptide-containing fractions are identified by conductivity, O.D.$_{276}$, HPLC and polyacrylamide gel electrophoresis. The fractions are then pooled.

An equal volume of acetic acid-urea solution is added to the pooled fractions. The material is then applied to a column containing S Sepharose® resin in acetic acid-urea sized to accommodate 50 g of protein per liter of resin. The flow rate is 0.02 1/cm$^2$/hr. The bGRF precursor fractions are eluted by a linear gradient of 0.25 M sodium chloride in acetic acid-urea. Fractions of 0.1 column volume are collected. The fractions are analyzed as before and the bGRF precursor-containing fractions are pooled.

A Sephadex® G-15 (Pharmacia) column is prepared in 0.02 M glycine, pH 2.5 with a column volume five times the volume of the previously pooled fractions. Fractions containing the O.D.$_{276}$ peak are isolated.

A column containing SP20SS resin (Sephabeads, Mitsubishi Chemical, Tokyo) in 10% acetonitrile-0.02 M glycine, pH 2.5, is then prepared. The pooled bGRF precursor-containing solution is made 10% in acetonitrile and added to the column at a flow rate of 1.5-2 column volumes per hour. The column is washed with 2 column volumes of the acetonitrile-glycine buffer. The bGRF precursor is eluted by a gradient formed by three column volumes of 10% acetonitrile-0.02 M glycine mixed with three column volumes 50% acetonitrile-0.02 M glycine. Fractions of 0.1 column volume are collected and assayed for the bGRF precursor.

The bGRF precursor-containing material is then chromatographed over a Sephadex® G15 column equilibrated in 0.25 M acetic acid. The O.D.$_{276}$ peak is then isolated and lyophilized until further use.

**Claims**

1.   A DNA compound which comprises an isolated DNA sequence encoding SEQ ID No 4.

2. The isolated DNA sequence of Claim 1 which is SEQ ID No 3.

3. A DNA compound which comprises an isolated DNA sequence which is SEQ ID No 1.

4. A polypeptide compound of the formula SEQ ID No. 4.

5. A polypeptide compound of the formula SEQ ID No. 6.

6. A pharmaceutical composition comprising as the active agent a polypeptide compound or a pharmaceutically acceptable acid or carboxylic acid addition salt thereof, wherein said polypeptide compound has the formula SEQ ID No. 4 and a pharmaceutically acceptable solid or liquid carrier.

**Claims for the following Contracting State : ES**

1. A method of producing the polypeptide compound of the formula SEQ ID No. 4 in a recombinant host cell which comprises:
   (1) transforming the host cell with a recombinant vector which comprises:
      (a) a transcription and translation activating sequence; and
      (b) the DNA compound of SEQ ID No. 3 positioned for expression from the activating sequence; and
   (2) culturing the host cell transformed in step (1) under conditions that allow for gene expression.

2. A method of producing the polypeptide compound of the formula SEQ ID No. 6 in a recombinant host cell which comprises:
   (1) transforming the host cell with a recombinant vector which comprises:
      (a) a transcription and translation activating sequence; and
      (b) the DNA compound of SEQ ID No. 5 positioned for expression from the activating sequence; and
   (2) culturing the host cell transformed in step (1) under conditions that allow for gene expression.

3. A method for inducing growth hormone release in an animal which comprises administering an effective amount of a polypeptide compound which is SEQ ID No 4 and the pharmaceutically acceptable acid or carboxylic acid addition salts thereof.

**Claims for the following Contracting State : GR**

1. A method of producing the polypeptide compound of the formula SEQ ID No. 4 in a recombinant host cell which comprises:
   (1) transforming the host cell with a recombinant vector which comprises:
      (a) a transcription and translation activating sequence; and
      (b) the DNA compound of SEQ ID No. 3 positioned for expression from the activating sequence; and
   (2) culturing the host cell transformed in step (1) under conditions that allow for gene expression.

2. A method of producing the polypeptide compound of the formula SEQ ID No. 6 in a recombinant host cell which comprises:
   (1) transforming the host cell with a recombinant vector which comprises:
      (a) a transcription and translation activating sequence; and
      (b) the DNA compound of SEQ ID No. 5 positioned for expression from the activating sequence; and
   (2) culturing the host cell transformed in step (1) under conditions that allow for gene expression.

3. A method for inducing growth hormone release in an animal which comprises administering an effective amount of a polypeptide compound which is SEQ ID No 4 and the pharmaceutically acceptable acid or carboxylic acid addition salts thereof.

4. A DNA compound which comprises an isolated DNA sequence encoding SEQ ID No. 4.

5. The isolated DNA sequence of Claim 1 which is SEQ ID No.3.

6. A DNA compound which comprises an isolated DNA sequence which is SEQ ID No. 1.

# FIG. I

European Patent
Office

# EUROPEAN SEARCH REPORT

Application Number

EP     92 30 3465
PAGE 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 212 531 (SYNTEX (USA) INC.) <br> * Whole document * <br> --- | 1-6 | C12N15/16 <br> A61K37/43 <br> C07K15/00 |
| Y | EP-A-0 134 085 (THE SALK INSTITUTE FOR BIOLOGICAL STUDIES) <br> * Whole document * <br> --- | 1-6 | |
| A | EP-A-0 317 387 (INSTITUT NATIONAL DE LA RECHERCHE AGRONOMIQUE) <br> * Whole document, in particular column 1 lines 43 - 54 * <br> --- | 1-6 | |
| Y | FASEB (FED. AM. SOC. FOR EXP. BIOL.) JOURNAL vol. 4, no. 4, 28 February 1990, WASHINGTON, US page A1170; <br> D.P. SMITH ET AL.: 'Expression and biological activity of bovine groth hormone releasing factor' <br> * Abstract * <br> --- | 1-6 | |
| Y | NATURE vol. 314, no. 6010, 4 April 1985, LONDON GB pages 464 - 467; <br> K.E. MAYO ET AL.: 'Characterization of complementary DNA and genomic clones encoding the precursor to rat hypothalamic growth hormone-releasing factor' <br> * Whole article * <br> --- | 1-6 | TECHNICAL FIELDS SEARCHED (Int. Cl.5 ) <br><br> C12N <br> C07K |
| A | PROCEEDINGS OF THE NATIONAL ACADEMY OF SCIENCES OF USA. vol. 80, no. 14, July 1983, WASHINGTON US pages 4311 - 4314; <br> U. GUBLER ET AL.: 'Cloning and sequence analysis of cDNA for the precursor of human growth hormone-releasing factor, somatocrinin' <br> * Whole article * <br> --- | 1-6 | |
| | -/-- | | |

The present search report has been drawn up for all claims

| Place of search | Date of completion of the search | Examiner |
|---|---|---|
| BERLIN | 04 AUGUST 1992 | JULIA P. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)

**European Patent Office**

## EUROPEAN SEARCH REPORT

Application Number

EP 92 30 3465
PAGE2

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | PROCEEDINGS OF THE NATIONAL ACADEMY OF SCIENCES OF USA. vol. 82, no. 1, January 1985, WASHINGTON US pages 63 - 67; K.E. MAYO ET AL.: 'Gene encoding human growth hormone-releasing factor precursor. Structure, sequence and chromosomal assignment' * Whole document * | 1-6 | |
| Y | EP-A-0 213 472 (SYNTEX (USA) INC.) * Page 23 line 30 - page 28 line 20 and page 58 example 8 * | 1-6 | |
| P,A | EP-A-0 459 747 (ELI LILLY AND CO.) * Whole document * | 1-6 | |
| | | | TECHNICAL FIELDS SEARCHED (Int. Cl.5) |

The present search report has been drawn up for all claims

| Place of search | Date of completion of the search | Examiner |
|---|---|---|
| BERLIN | 04 AUGUST 1992 | JULIA P. |

## SEQUENCE LISTING

(1) GENERAL INFORMATION:

    (i) APPLICANT:

            Eli Lilly and Company

    (ii) TITLE OF INVENTION: PRECURSOR FORM OF BOVINE GROWTH HORMONE
         RELEASING FACTOR AND RELATED DNA COMPOUNDS

    (iii) NUMBER OF SEQUENCES: 6

    (iv) CORRESPONDENCE ADDRESS:
        (A) ADDRESSEE:  Mr. C. Mark Hudson
        (B) STREET:     Erl Wood Manor
        (C) CITY:       Windlesham
        (D) STATE:      Surrey
        (E) COUNTRY:    Grande Bretagne
        (F) ZIP:        GU20 6PH

    (v) ATTORNEY/AGENT INFORMATION:
        (A) NAME:  Mr. C. Mark Hudson
        (B) REGISTRATION NUMBER:  307
        (C) REFERENCE/DOCKET NUMBER: X-8377

    (vi) TELECOMMUNICATION INFORMATION:
        (A) TELEPHONE: (0276) 78441
        (B) TELEFAX:   (0276) 78306

(2) INFORMATION FOR SEQ ID NO:1:

    (i) SEQUENCE CHARACTERISTICS:
        (A) LENGTH: 132 base pairs
        (B) TYPE: nucleic acid
        (C) STRANDEDNESS: double
        (D) TOPOLOGY: linear

    (ii) MOLECULE TYPE: cDNA

    (ix) FEATURE:
        (A) NAME/KEY: CDS
        (B) LOCATION: 1..132

    (xi) SEQUENCE DESCRIPTION: SEQ ID NO:1:

```
TAC GCA GAT GCC ATC TTC ACT AAC AGC TAC CGG AAG GTT CTG GGC CAG      48
Tyr Ala Asp Ala Ile Phe Thr Asn Ser Tyr Arg Lys Val Leu Gly Gln
 1               5                  10                  15

CTG TCT GCC CGC AAG CTA CTC CAG GAT ATC ATG AAC AGG CAG CAG GGA      96
Leu Ser Ala Arg Lys Leu Leu Gln Asp Ile Met Asn Arg Gln Gln Gly
             20                  25                  30

GAG AGA AAC CAG GAG CAA GGA GCA AAG GTA CGG CTT                      132
Glu Arg Asn Gln Glu Gln Gly Ala Lys Val Arg Leu
         35                  40
```

(2) INFORMATION FOR SEQ ID NO:2:

    (i) SEQUENCE CHARACTERISTICS:
        (A) LENGTH: 44 amino acids
        (B) TYPE: amino acid
        (D) TOPOLOGY: linear

    (ii) MOLECULE TYPE: protein

(xi) SEQUENCE DESCRIPTION: SEQ ID NO:2:

```
Tyr Ala Asp Ala Ile Phe Thr Asn Ser Tyr Arg Lys Val Leu Gly Gln
 1               5                  10                  15

Leu Ser Ala Arg Lys Leu Leu Gln Asp Ile Met Asn Arg Gln Gln Gly
            20                  25                  30

Glu Arg Asn Gln Glu Gln Gly Ala Lys Val Arg Leu
            35                  40
```

(2)  INFORMATION FOR SEQ ID NO:3:

     (i)  SEQUENCE CHARACTERISTICS:
        (A)  LENGTH: 228 base pairs
        (B)  TYPE: nucleic acid
        (C)  STRANDEDNESS: double
        (D)  TOPOLOGY: linear

    (ii)  MOLECULE TYPE: cDNA

    (ix)  FEATURE:
        (A)  NAME/KEY: CDS
        (B)  LOCATION: 1..228

(xi)  SEQUENCE DESCRIPTION: SEQ ID NO:3:

```
TAC GCA GAT GCC ATC TTC ACT AAC AGC TAC CGG AAG GTT CTG GGC CAG   48
Tyr Ala Asp Ala Ile Phe Thr Asn Ser Tyr Arg Lys Val Leu Gly Gln
 1               5                  10                  15

CTG TCT GCC CGC AAG CTA CTC CAG GAT ATC ATG AAC AGG CAG CAG GGA   96
Leu Ser Ala Arg Lys Leu Leu Gln Asp Ile Met Asn Arg Gln Gln Gly
            20                  25                  30

GAG AGA AAC CAG GAG CAA GGA GCA AAG GTA CGG CTT GGC CGT CAG GTG  144
Glu Arg Asn Gln Glu Gln Gly Ala Lys Val Arg Leu Gly Arg Gln Val
            35                  40                  45

GAC GGC GTG TGG ACA GAC CAA CAG CAG ATG GCA CTG GAG AGC ACC TTG  192
Asp Gly Val Trp Thr Asp Gln Gln Gln Met Ala Leu Glu Ser Thr Leu
        50                  55                  60

GTG AGC CTG CTG CAG GAG CGC AGG AAT TCC CAA GGA                  228
Val Ser Leu Leu Gln Glu Arg Arg Asn Ser Gln Gly
65                  70                  75
```

(2) INFORMATION FOR SEQ ID NO:4:

    (i) SEQUENCE CHARACTERISTICS:
       (A) LENGTH: 76 amino acids
       (B) TYPE: amino acid
       (D) TOPOLOGY: linear

    (ii) MOLECULE TYPE: protein

    (xi) SEQUENCE DESCRIPTION: SEQ ID NO:4:

```
Tyr Ala Asp Ala Ile Phe Thr Asn Ser Tyr Arg Lys Val Leu Gly Gln
 1               5                  10                  15

Leu Ser Ala Arg Lys Leu Leu Gln Asp Ile Met Asn Arg Gln Gln Gly
            20                  25                  30

Glu Arg Asn Gln Glu Gln Gly Ala Lys Val Arg Leu Gly Arg Gln Val
            35                  40                  45

Asp Gly Val Trp Thr Asp Gln Gln Gln Met Ala Leu Glu Ser Thr Leu
        50                  55                  60

Val Ser Leu Leu Gln Glu Arg Arg Asn Ser Gln Gly
 65                  70                  75
```

(2) INFORMATION FOR SEQ ID NO:5:

    (i) SEQUENCE CHARACTERISTICS:
       (A) LENGTH: 318 base pairs
       (B) TYPE: nucleic acid
       (C) STRANDEDNESS: double
       (D) TOPOLOGY: linear

    (ii) MOLECULE TYPE: cDNA

    (ix) FEATURE:
       (A) NAME/KEY: CDS
       (B) LOCATION: 1..318

    (xi) SEQUENCE DESCRIPTION: SEQ ID NO:5:

```
ATG CTG CTC TGG GTG TTC TTC CTC GTG ACC CTC ACC CTC AGC AGC GGC  48
Met Leu Leu Trp Val Phe Phe Leu Val Thr Leu Thr Leu Ser Ser Gly
 1               5                  10                  15

TCC CAC GGT TCC CTG CCT TCC CAG CCT CTC AGG ATT CCA CGG TAC GCA  96
Ser His Gly Ser Leu Pro Ser Gln Pro Leu Arg Ile Pro Arg Tyr Ala
            20                  25                  30
```

```
GAT GCC ATC TTC ACT AAC AGC TAC CGG AAG GTT CTG GGC CAG CTG TCT    144
Asp Ala Ile Phe Thr Asn Ser Tyr Arg Lys Val Leu Gly Gln Leu Ser
        35                  40                  45

GCC CGC AAG CTA CTC CAG GAT ATC ATG AAC AGG CAG CAG GGA GAG AGA    192
Ala Arg Lys Leu Leu Gln Asp Ile Met Asn Arg Gln Gln Gly Glu Arg
        50                  55                  60

AAC CAG GAG CAA GGA GCA AAG GTA CGG CTT GGC CGT CAG GTG GAC GGC    240
Asn Gln Glu Gln Gly Ala Lys Val Arg Leu Gly Arg Gln Val Asp Gly
    65                  70                  75                  80

GTG TGG ACA GAC CAA CAG CAG ATG GCA CTG GAG AGC ACC TTG GTG AGC    288
Val Trp Thr Asp Gln Gln Gln Met Ala Leu Glu Ser Thr Leu Val Ser
                85                  90                  95

CTG CTG CAG GAG CGC AGG AAT TCC CAA GGA                            318
Leu Leu Gln Glu Arg Arg Asn Ser Gln Gly
            100                 105
```

(2)  INFORMATION FOR SEQ ID NO:6:

    (i)  SEQUENCE CHARACTERISTICS:
        (A)  LENGTH: 106 amino acids
        (B)  TYPE: amino acid
        (D)  TOPOLOGY: linear

    (ii)  MOLECULE TYPE: protein

    (xi)  SEQUENCE DESCRIPTION: SEQ ID NO:6:

```
Met Leu Leu Trp Val Phe Phe Leu Val Thr Leu Thr Leu Ser Ser Gly
 1               5                  10                  15

Ser His Gly Ser Leu Pro Ser Gln Pro Leu Arg Ile Pro Arg Tyr Ala
            20                  25                  30

Asp Ala Ile Phe Thr Asn Ser Tyr Arg Lys Val Leu Gly Gln Leu Ser
        35                  40                  45

Ala Arg Lys Leu Leu Gln Asp Ile Met Asn Arg Gln Gln Gly Glu Arg
    50                  55                  60

Asn Gln Glu Gln Gly Ala Lys Val Arg Leu Gly Arg Gln Val Asp Gly
    65                  70                  75                  80

Val Trp Thr Asp Gln Gln Gln Met Ala Leu Glu Ser Thr Leu Val Ser
                85                  90                  95

Leu Leu Gln Glu Arg Arg Asn Ser Gln Gly
            100                 105
```